# EUROPÄISCHE PATENTANMELDUNG

(11) **EP 1 239 408 A2**
(43) Veröffentlichungstag der Anmeldung: **11.09.2002**
(21) Anmeldenummer: 01127126.9
(22) Anmeldetag: 14.11.2001
(51) Int. Cl.: G06K 19/04

(54) **Übermittlungssystem für medizinische Informationen**

(30) Priorität: 16.11.2000 DE 10056886
(71) Anmelder: Edlhuber, Christian, 82481 Mittenwald (DE)
(72) Erfinder: Edlhuber, Christian, 82481 Mittenwald (DE)

(57) **Zusammenfassung**

Beschrieben wird ein Informationsvermittlungssystem, mit welchem wenigstens eine erste, fachlich autorisierte Person fach- und/oder personenbezogene Daten, welche wenigstens einer anderen Person zugeordnet sind, in codierter und von dieser Person mitführbarer Form aufzeichnen und festhalten kann, um es dieser wenigstens einen anderen Person und/oder wenigstens einer weiteren, ebenfalls fachlich autorisierten Person zu ermöglichen, ohne persönliche Anwesenheit der ersten autorisierten Person anhand der fach- und/oder personenbezogenen Daten Handlungsentscheidungen zu treffen. Die Daten sind auf einem von der Person mitführbaren Datenträger (2) in unverlierbarer Form hinterlegt, wobei die Daten auf dem Datenträger (2) durch wenigstens eine, bevorzugt mehrere das Material des Datenträger durchlässig machende Ausnehmungen in Form von umfangsseitig ausgebildeten, zahnförmigen Aussparungen (20a,20b,22,24) codiert sind.

## Beschreibung

Die Erfindung betrifft ein Informationsvermittlungssystem, die Verwendung eines derartigen Informationsvermittlungssystems, sowie ein Verfahren zur Informationsvermittlung und -auswertung.

Der Gegenstand der vorliegenden Erfindung bzw. die der vorliegenden Erfindung zugrunde liegende Problematik sei nachfolgend anhand der Probleme und Nachteile geschildert, mit welchen Zahnärzte in zunehmenden Maße konfrontiert sind:

Aufgrund der bestehenden Konkurrenzsituation unter den Krankenkassen war es lange üblich, daß die Kassen ihren Mitgliedern selbst beschaffte zahnärztliche Versorgungen bzw. außervertragliche Leistungen weitestgehend bezahlt oder teilweise ganz erheblich bezuschußt haben. Ab 1993 sind neue Gesetze in Kraft getreten, welche diese zuletzt mehr und mehr ausufernde Leistungsausschüttung unterbunden haben. Die Krankenkassen dürfen seither nur noch das leisten, wozu sie nach dem Gesetz unmittelbar verpflichtet sind, so daß der Leistungskatalog der Krankenkassen nur noch diejenigen Leistungen umfaßt, zu denen die Kassen durch Gesetz bzw. Verträge verpflichtet sind.

Dies bedeutet, daß ein Zahnarzt mehr und mehr vor dem Problem steht, dem Patienten vor Augen zu führen, daß die gesetzliche Krankenversicherung nur einen eng umgrenzten Leistungskatalog beinhaltet, da dies vom Gesetzgeber so gewollt ist. Ein Patient, der mehr Leistung haben möchte, als die gesetzliche Krankenversicherung zur Verfügung stellt, muß sich der Tatsache bewußt sein, daß er diese über den Leistungskatalog hinausgehende Leistungen dann selbst bezahlen muß. Die Kassen werden nämlich stets behaupten, daß die grundlegende bzw. notwendige zahnmedizinische Versorgung nach wie vor sichergestellt ist; dem Zahnarzt bleibt es dann überlassen, dies im Einzelfall zu widerlegen.

Der Zahnarzt steht somit vor dem Problem, seinem Patienten einerseits eine optimale Behandlung zukommen zu lassen, es jedoch andererseits dem Patienten klarzumachen, daß diese für ihn optimale Behandlung mehr oder weniger weit über das hinausgeht, was die gesetzliche Kasse zu erstatten gewillt ist. Dies bedingt einen Umdenk- und Lernprozeß sowohl beim Zahnarzt, als auch insbesondere beim Patienten.

Es entspricht nämlich die nunmehr gültige oder praktizierte Vorgehensweise quasi der Übertragung einer in anderen Branchen üblichen Vorgehensweise auf den (Zahn)arztbereich, nämlich z.B. ein Handwerker kalkuliert seinen Kostenvoranschlag (der z.B. einem Heil- und Kostenplan der Krankenkasse im medizinischen Bereich entsprechen würde) nach, weil er wegen "unvorhersehbarer" Schwierigkeiten länger gebraucht hat. Diskussionen mit dem Auftraggeber (hier: Patient) sind vorprogrammiert, auch wenn der Auftragnehmer (hier: Arzt) noch so viele Gründe für die Notwendigkeit der über das von den Kassen vorgegebene Maß hinausführenden Behandlung anführt.

Neben diesem Problem der Abrechnung und Vergütung von erbrachten Leistungen besteht sowohl bei Zahnärzten, jedoch auch bei anderen Ärzten, Therapeuten, Medizintechnikern, Dentalhygienikern etc. die Notwendigkeit, im Zuge einer Behandlung oder nach erfolgreicher Beendigung derselben dem Patienten Informationen mitzugeben, wie beispielsweise eine soeben erfolgreich abgeschlossene Behandlung seitens des Patienten eigenverantwortlich zuhause unterstützt oder fortgeführt werden kann/muß, um den angestrebten oder bereits erreichten Heilungs- oder Therapieeffekt zu erzielen oder zu erhalten.

Beispielsweise auf dem Gebiet der Dentalmedizin muß der Zahnarzt oder Dentalhygieniker seinem Patienten gewisse Empfehlungen oder Weisungen betreffend jeweils optimal für den jeweiligen Patienten bzw. dessen Therapiebild geeignete Zahn- oder Mundpflegemittel mitgeben. Hält man sich das für Laien schwierig zu überschauende Angebot an Zahn- und Mundpflegeartikeln beispielsweise in Drogeriemärkten vor Augen, erkennt man das Problem, daß es für einen Patienten oftmals schwierig ist, beispielsweise die ihm vom Zahnarzt empfohlene Zahncreme aus dem Angebot herauszufinden. Weitaus gravierender wird dieses Problem, wenn der Zahnarzt darauf verzichtet hat, dem Patienten eine Zahncreme mit einem bestimmten Produktnamen zu empfehlen, sondern nur eine Zahncreme mit einem bestimmten Wirkstoffkomplex empfohlen hat. Für einen Laien ist es praktisch unmöglich, aus der großen zur Verfügung stehenden Produktpalette dann das für ihn optimale Produkt herauszufinden. Aber auch wenn der Zahnarzt ein ganz konkretes Produkt empfohlen hat, kann sich das Problem ergeben, daß gerade dieses Produkt momentan im Drogeriemarkt oder einem sonstigen Geschäft nicht zur Verfügung steht. Der Kunde/Patient ist dann entweder dazu gezwungen, ein anderes Geschäft oder einen anderen Drogeriemarkt aufzusuchen oder er sieht sich nach einem Produkt eines anderen Herstellers mit gleichem oder zumindest annähernd gleichem Wirkstoffkomplex um. Dann wiederum ergibt sich für ihn als medizinischen Laien das Problem, ein derartiges Produkt aus der großen Produktpalette herauszufinden.

Es sei nochmals festgehalten, daß diese Problematik zwar anhand der sich auf dem Gebiet der Zahn- oder Mundpflege ergebenden Probleme erläutert wurde, sie jedoch gleichermaßen auch auf anderen medizinischen Gebieten besteht. Insbesondere dann, wenn der Arzt oder Therapeut oder auch Medizintechniker seinem Patienten ein bestimmtes Produkt und/oder bestimmte Therapiehilfsmittel empfiehlt und der Patient diese Produkte oder Therapiehilfsmittel in Eigenregie beispielsweise in einem Drogeriemarkt oder dergleichen erwerben kann, also keine konkrete Verschreibung in Form eines Rezeptes vorhanden ist, welches in eindeutiger Weise in einer Apotheke eingelöst wird, können sich die genannten Probleme ergeben, welche zu einer Verunsicherung des Patienten, zu einer nicht optimalen Therapieunterstützung oder Nachtherapie oder zu anderen Problemen führen. Beispiele hierfür seien beispielsweise ärztliche Empfehlungen in bestimmten orthopädischen Fällen (Stützbandagen, Gymnastikhilfsmittel etc.), der Augenheilkunde (beispielsweise Pflegemittel für bestimmte Kontaktlinsentypen), Kinderheilkunde (Empfehlungen für bestimmte Pflegecremes, hautverträgliche Windeln etc.) und dergleichen.

Es besteht somit Bedarf an einem Hilfsmittel, mit welchem die vom jeweiligen Arzt oder Therapeut gegebene Empfehlung vom Patienten aus der Praxis heraus mitgenommen und dann an anderer Stelle entsprechend ausgewertet werden kann, um an dieser anderen Stelle, beispielsweise dem Kaufort für die vom Arzt empfohlenen Produkte, die notwendigen Informationen zur Verfügung zu haben.

Die vorliegende Erfindung hat es sich zur Aufgabe gemacht, diese Notwendigkeit zu erfüllen und schafft ein Informationsvermittlungssystem, mit welchem wenigstens eine erste, fachlich autorisierte Person fach- und/oder personenbezogene Daten, welche wenigstens einer anderen Person zugeordnet sind, in codierter und von dieser Person mitführbarer Form aufzeichnen und festhalten kann, um es dieser wenigstens einen anderen Person und/oder wenigstens einer weiteren, ebenfalls fachlich autorisierten Person zu ermöglichen, ohne persönliche Anwesenheit der ersten autorisierten Person anhand der fach- und/oder personenbezogenen Daten Handlungsentscheidungen zu treffen.

Die vorliegende Erfindung schafft weiterhin die Verwendung eines derartigen Informationsvermittlungssystems zur Abspeicherung fach- und/oder personenorientierter Daten in codierter Form und zur Mitgabe der so gespeicherten Daten an eine diese Daten betreffende Person, welche diese Daten dann für sich selbst oder durch eine andere fachlich autorisierte Person auswerten kann.

Schließlich schafft die vorliegende Erfindung ein Verfahren zur Informationsübermittlung und -auswertung, bei welchem fach- und/oder personenorientierte Daten durch eine fachlich autorisierte Person in codierter Form abgespeichert werden und die so abgespeicherten Daten einer diese Daten betreffenden Person mitgegeben werden, welche dann diese Daten für sich selbst oder durch eine andere fachlich autorisierte Person auswertet.

Durch den Gegenstand der vorliegenden Erfindung wird eine Möglichkeit geschaffen, den Dialog zwischen Arzt und Patient, Therapeut und Patient etc. bzw. die Unterstützung des Arztes oder Therapeuten für seinen Patienten aus der Praxis heraus zu verlagern, um dem Patienten eine Möglichkeit zu geben, Therapiehinweise oder -empfehlungen in unverlierbarer Form mitzuführen. Die sich hieraus ergebenden Vorteile, sowie weitere, der vorliegenden Erfindung inhärente Vorteile werden sich aus der nachfolgenden Beschreibung noch näher ergeben.

Bevorzugt sind die fach- und/oder personenbezogenen Daten auf einem von der Person mitführbaren Datenträger in unverlierbarer Form hinterlegt, wobei weiterhin bevorzugt der Datenträger in Form eines flächigen Trägerkörpers ausgebildet ist.

Weiterhin weist bevorzugt der Träger festgelegte Abmessungen auf, insbesondere die Abmessungen einer üblichen Schutzhülle für Scheckkarten. Durch diese vorteilhaften oder bevorzugten Ausgestaltungsformen des Datenträgers oder Trägerkörpers wird dem Benutzer ein benutzerfreundliches System zur Verfügung gestellt, welches die Daten in unverlierbarer Form enthält und aufgrund seiner Abmessungen beispielsweise in vorhandene Aufbewahrungssysteme in Brieftaschen, Kraftfahrzeugen etc. paßt.

Die Daten sind auf dem Trägerkörper auf eine Mehrzahl von Arten in unverlierbarer Form hinterlegbar oder hinterlegt, Beispielsweise können die Daten auf dem Trägerkörper mittels wenigstens einem im oder auf dem Trägerkörper integrierten Chip und/oder Magnetstreifen und/oder Transponder gespeichert sein.

Für Chips, Magnetstreifen oder Transponder sind entsprechende Schreib/Lesegeräte hoher Quailtät und Zuverlässigkeit preiswert verfügbar.

Weiterhin können die Daten auf dem Trägerkörper durch wenigstens eine, bevorzugt mehrere das Material des Trägerkörpers durchtretende Ausnehmungen codiert sein. Im Gegensatz zu Chips, Magnetstreifen oder Transpondern erfolgt bei dieser Ausgestaltungsform die Codierung der Daten auf mechanischem Weg, nämlich durch die Ausnehmungen, welche das Material des Trägerkörpers durchtreten. Auch hierzu sind entsprechende Stanz- und Lesegeräte verfügbar.

Die Ausnehmungen können hierbei z.B. in Form von umfangsseitig am Trägerkörper ausgebildeten, zahnförmigen Aussparungen ausgebildet sein, sie können in Form von am Umfangsrand des Trägerkörpers in einem bestimmten Code-Muster abwechselnd ausgebildeten lichtdurchlässigen und lichtundurchlässigen Bereichen oder sie können in Form von in der Fläche des Trägerkörpers ausgebildeten Durchbrechungen gebildet sein. In allen Fällen ist es möglich, entsprechende Daten auf dem Trägerkörper durch Aussparungen oder Ausnehmungen zu hinterlegen.

Bevorzugt sind hierbei die Ausnehmungen in zweckorientierten Gruppen und/oder Reihen und/oder Spalten angeordnet, um die Codierung und auch Decodierung gegebenenfalls erleichtern zu können.

Die Ausnehmungen können weiterhin bevorzugt durch Herausbrechen und/oder Herausstanzen und/oder Herausschneiden aus dem Material des Trägerkörpers bildbar sein, wobei weiterhin diejenigen Stellen, an welchen die Ausnehmungen aus bildbar sind, durch Markierungen in Form von Vorstanzungen und/oder Linien und/oder Farbflächen vorgegeben sind. Hierdurch wird es möglich, auch ohne entsprechend ausgestattete Stanzgeräte die Ausnehmungen von Hand durch ein entsprechendes Werkzeug auszubilden.

In den als Schutzhülle ausgebildeten Trägerkörper kann gemäß einer weiterhin bevorzugten Ausgestaltungsform eine zum System gehörende Karte einführbar und hierin aufbewahrbar sein. Dies entspricht zwar der zwar an sich bekannten Kombination aus Scheck- oder Telefonkarte mit zugehöriger Schutzhülle, bietet jedoch im Rahmen des erfindungsgemäßen Systems den zusätzlichen erheblichen Vorteil, daß auf der Karte im Bedarf zusätzliche fachbezogenen Daten, welche der anderen Person zugeordnet sind, abspeicherbar sind, wobei diese Daten wiederum auf einem in der Karte integrierten Chip und/oder Magnetstreifen und/oder Transponder abgespeichert werden können.

Wie sich aus der nachfolgenden Beschreibung noch ergeben wird, kann hierdurch das erfindungsgemäße Informationsvermittlungssystem nicht nur zur alleinigen Informationsweitergabe verwendet werden, sondern auch zur Abrechnung vor Ort, z.B. von seiten eines Arztes oder Therapeutes erbrachten Leistungen, wobei durch das erfindungsgemäße System ein gewisser Lerneffekt seitens des Patienten insofern auftritt, als ihm aufgezeigt wird, welchen Kostenrahmen im Zuge der Behandlung die Kasse zu übernehmen gewillt ist und welche darüber hinaus gehenden Leistungen er als Patient selbst bezahlen muß.

Weitere Einzelheiten, Aspekte und Vorteile der vorliegenden Erfindung ergeben sich aus der nachfolgenden Beschreibung von Ausführungsformen anhand der Zeichnung.

Es zeigt:
Fig. 1 eine Ansicht eines Trägerkörpers gemäß einer ersten Ausführungsform der vorliegenden Erfindung;
Fig. 2 eine Fig. 1 entsprechende Ansicht einer zweiten Ausführungsform der vorliegenden Erfindung;
Fig. 3 die Ansicht einer Kombination aus Trägerkörper gemäß Fig. 1 mit einer zugehörigen Karte;
Fig. 4 eine Ansicht einer dritten Ausführungsform eines im erfindungsgemäßen Informationsvermittlungssystem verwendbaren Trägerkörpers; und
Fig. 5 ein Flußdiagramm zur Erläuterung eines mit dem erfindungsgemäßen Informationsvermittlungssystem realisierbaren Ablaufs.

In der nachfolgenden Beschreibung bevorzugter Ausführungsformen der Erfindung, welche gleichwohl als rein illustrativ und nicht einschränkend zu verstehen sind, werden gleiche oder einander entsprechende Teile mit gleichen Bezugszeichen versehen; eine wiederholte ausführliche Beschreibung dieser Teile erfolgt nicht.

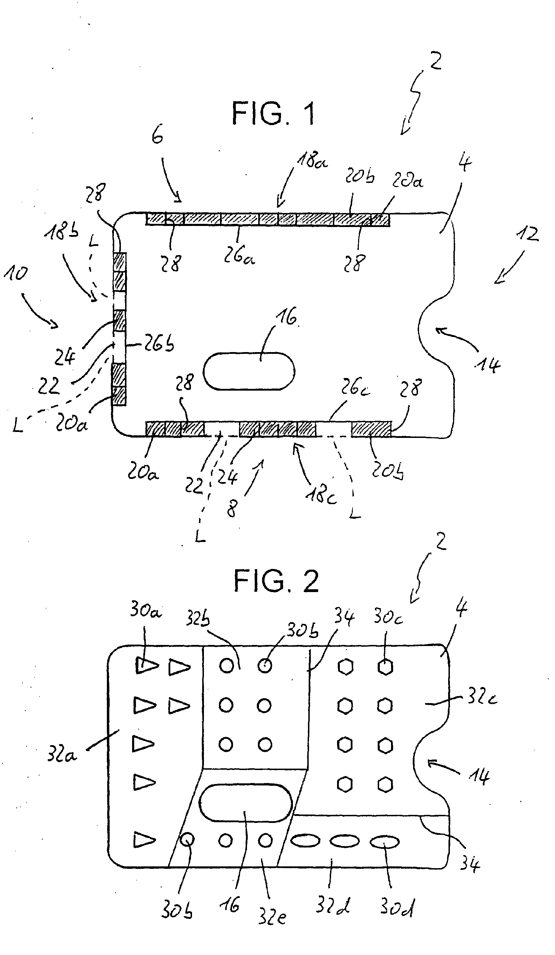

Fig. 1 zeigt eine erste Ausführungsform eines erfindungsgemäßen Informationsvermittlungssystems bzw. eines hierzu gehörenden Datenträgers 2. Der Datenträger 2 ist in Form eines flächigen Trägerkörpers 4 ausgebildet, der im gleiche oder einander entsprechende Teile mit gleichen Bezugszeichen versehen; eine wiederholte ausführliche Beschreibung dieser Teile erfolgt nicht.

Fig. 1 zeigt eine erste Ausführungsform eines erfindungsgemäßen Informationsvermittlungssystems bzw. eines hierzu gehörenden Datenträgers 2. Der Datenträger 2 ist in Form eines flächigen Trägerkörpers 4 ausgebildet, der im dargestellten, besonders bevorzugten Ausführungsbeispiel die Form einer üblichen Schutzhülle ("cover") für Scheck- oder Kontokarten oder dergleichen hat. Derartige Schutzhüllen oder cover weisen festgelegte Abmessungen auf und bestehen im wesentlichen aus zwei flächigen Hüllenteilen, welche an zwei gegenüberliegenden Langseiten 6 und 8 und einer Schmalseite 10 miteinander verbunden sind. An der der Schmalseite 10 gegenüberliegenden Schmalseite 12 ist zwischen den beiden Hüllenteilen ein durchgehender Spalt oder Schlitz gebildet, durch welchen in den Trägerkörper 4 eine Karte entsprechender Abmessung eingeschoben werden kann. Zum leichteren Herausziehen der Karte aus dem Trägerkörper 4 ist im Bereich der Schmalseite 12 in bekannter Weise eine Ausnehmung 14 ausgebildet, an der eine in den Trägerkörper 4 eingeschobene Karte ergriffen werden kann. Weiterhin ist zumindest in einem der beiden Hüllenteile in bekannter Weise eine weitere Ausnehmung 16 ausgebildet, durch welche eine korrekt in den Trägerkörper 4 eingeschobene Karte identifiziert werden kann.

Der Trägerkörper 4 (cover) dient im erfindungsgemäßen Informationsvermittlungssystem zur Aufnahme, d.h. Abspeicherung von fach- und/oder personenbezogenen Daten. ("Fachbezogen" bedeuten im Rahmen der vorliegenden Erfindung Daten, welche bestimmte Sachverhalte, Hinweise, Empfehlungen für Therapien oder Therapiehilfsmittel, etc. betreffen, also Daten, welche ein bestimmtes - z.B. medizinisches - Fachgebiet betreffen. Insofern ist "fachbezogen" gleichzusetzen mit "sachbezogen".)

Hierzu weist in der Ausführungsform gemäß Fig. 1 der Trägerkörper 4 zumindest an einer Seite, bevorzugt jedoch an den beiden Längsseiten 6 und 8 und auch der Schmalseite 10 Codierbereiche 18a, 18b und 18c auf. Diese Codierbereiche 18a bis 18c liegen in Form von nebeneinander liegenden, aus dem Material des Trägerkörpers 4 entfernbaren Abschnitten 20a und 20b vor, wobei im gezeigten Ausführungsbeispiel die Abschnitte 20a gegenüber den Abschnitten 20b geringere Größe haben können. Selbstverständlich können die einzelnen Abschnitte auch gleiche Größe haben. Weiterhin können die Abschnitte optional auch unterschiedliche Formgebung (dreieckig, halbkreisförmig etc.) haben.

Die Abschnitte 20a und 20b sind gemäß Fig. 1 entlang der Längsseite 6 im dortigen Codierbereich 18a, entlang der Längsseite 8 im Bereich des dortigen Codierbereiches 18c und entlang der Schmalseite 10 im dortigen Codierbereich 18b aufeinanderfolgend in einer bestimmten Sequenz oder Abfolge angeordnet.

Durch Entfernen einzelner Abschnitte 20a und/oder 20b aus den jeweiligen Codierbereichen 18a bis 18c, wie dies in Fig. 1 in den Codierbereichen 18b und 18c dargestellt ist, lassen sich in codierter, beispielsweise binärer Form, bestimmte Daten auf dem Trägerkörper 4 in unverlierbarer Form festlegen oder hinterlegen.

Das Entfernen einzelner Abschnitte 20a und 20b aus den jeweiligen Codierbereichen 18a bis 18c kann durch Herausbrechen, Herausstanzen oder Herausschneiden der jeweiligen Abschnitte 20a und/oder 20b aus dem Material des Trägerkörpers 4 erfolgen. Hierzu kann gemäß einer Vorgehensmöglichkeit der gesamte Datenträger 2 bzw. der Trägerkörper 4 in eine entsprechende Vorrichtung oder ein entsprechendes Gerät eingeschoben werden. Über eine mit dem Gerät verbundene Tastatur werden die entsprechenden Daten eingegeben und von dem Gerät in passende Codierungssequenzen umgesetzt, wonach dann selektiv betätigbare Stanzwerkzeuge die entsprechenden Abschnitte 20a und/oder 20b aus dem Material des Trägerkörpers 4 entfernen, so daß sich zahnförmige Aussparungen 22 bzw. zahnförmige Vorsprünge 24 in den Codierbereichen 18a bis 18c ergeben.

Eine weitere Vorgehensweise ist, die gewünschten Abschnitte 20a und/oder 20b in einem Gerät markieren zu lassen, daß heißt, anstelle selektiv betätigbarer Stanzwerkzeuge befinden sich in dem Gerät selektiv betätigbare Markierungsvorrichtungen, welche die zu entfernenden Abschnitte 20a und/oder 20b auf geeignete Weise markieren. Nach Herausziehen des Trägerkörpers 4 aus dem Gerät können dann die markierten Abschnitte 20a und/oder 20b herausgeschnitten oder herausgebrochen werden. Hierzu kann es vorteilhaft sein, Anschlußlinien 26a, 26b und 26c zwischen den Codierbereichen 18a, 18b und 18c mit dem Material des Trägerkörpers 4 und Verbindungslinien 28 zwischen den einzeln nen Abschnitten 20a und 20b bzw. zwischen dem Trägerkörper 4 und den Abschnitten 20a und 20b in Form von Vorstanzungen, das heißt Sollbruchstellen auszubilden.

Ein entsprechend geschultes Personal vorausgesetzt, können die Abschnitte 20a und/oder 20b auch direkt aus dem Trägerkörper 4 entfernt werden, d.h. ohne Schreib- oder Markiergerät.

Eine weitere bevorzugte Ausgestaltungsform des Trägerkörpers ist, die einzelnen Codierbereiche 18a, 18b und 18c nicht mit körperlich entfernten Aussparungen 22 zu versehen, sondern den Umfangsrand des Trägerkörpers 4 unversehrt, d.h. glatt zu belassen (wie in Fig. 1 durch die gestrichelten Linien L angedeutet) und anstelle der Ausparungen 22 lichtdurchlässige Abschnitte vorzusehen und anstelle der Vorsprünge 24 lichtundurchlässige Abschnitte. Herstellen lassen sich derartige lichtdurchlässige/lichtundurchlässige Codierungen z.B. mit einem PC mit entsprechendem Programm, einem Drucker und einem Laminiergerät, welches eine entsprechend bedruckte Folie auf den Trägerkörper auflaminiert.

Auch hiermit kann so eine maschinenlesbare Codierung geschaffen werden, wobei sich der Vorteil ergibt, daß aufgrund des glatten Umfangsrandes des Trägerkörpers 4 sich dieser nicht in Hosentaschen, Geldbörsen oder anderen Aufbewahrungsorten, an Schlüsselbunden etc. verhaken kann, wobei u. U. durch Wegbrechen oder Verbiegen der Vorsprünge 24 die Codierung unlesbar oder verändert werden könnte. Die Ablesung der codierten Nachricht erfolgt dann in Lesegeräten mit mehreren Lichtschranken, z.B. LED-Reihen mit gegenüberliegenden Photosensor-Reihen, zwischen denen dann der Rand oder die Ränder des Trägerkörpers 4 zu liegen kommt oder kommen.

Fig. 2 zeigt eine zweite Ausführungsform eines Datenträgers 2 bzw. Trägerkörpers 4. Analog zum Ausführungsbeispiel von Fig. 1 ist der Trägerkörper 4 als Aufnahmehülle für eine Scheck- und hier insbesondere Krankenkassenkarte ausgebildet. Anstelle der Codierbereiche 18a bis 18c im Bereich der Längsseiten 6 und 8 und der Schmalseite 10 weist in der Ausführungsform von Fig. 2 der Trägerkörper 4 Ausnehmungen 30a, 30b, 30c und 30d auf, welche das Material des Trägerkörpers 4 vollständig durchsetzen, das heißt, nicht wie im Falle der Ausnehmung 16 nur eine der beiden Schutzhüllenteile, sondern beide. Die Ausnehmungen 30a bis 30d können, müssen jedoch nicht in Gruppen reihen- und/oder spaltenförmig zusammengefaßt sein und können, müssen jedoch nicht unterschiedliche Formgebung haben.

Die Ausführungsmöglichkeit der Zusammenfassung in Gruppen und der Ausbildung der einzelnen Ausnehmungen in unterschiedlichen Formen ist in Fig. 2 dargestellt, das heißt, die Ausnehmungen 30a haben Dreiecksform und sind zu einer Gruppe 32a (2 Spalten, 2 Reihen) zusammengefaßt, die Ausnehmungen 30b haben Kreisform und sind in einer Gruppe 32b bzw. 32e (2 Spalten, 3 Reihen bzw. 1 Reihe) zusammengefaßt, die Ausnehmungen 30c haben Sechseckform und sind in einer Gruppe 32c (2 Spalten, 4 Reihen) zusammengefaßt und die Ausnehmungen 30d haben Ellipsenform und sind in einer Gruppe 32d (1 Reihe) zusammengefaßt. Die einzelnen Gruppen 32a bis 32e können auch durch auf dem Material des Trägerkörpers 4 aufgedruckte oder sonstwie aufgebrachte Trennlinien 34 sichtbar voneinander abgegrenzt werden, wobei die einzelnen Flächen der Gruppen 32a bis 32d darüber hinaus noch bei Bedarf unterschiedliche farbige Ausgestaltungen haben können.

Die Ausnehmungen 30a bis 30d können wie im Ausführungsbeispiel von Fig. 1 z.B. durch entsprechende Geräte oder Werkzeuge oder direkt von Hand im Trägerkörper 4 ausgebildet werden, um die notwendigen Daten in unverlierbarer codierter Form auf dem Trägerkörper 4 festzuhalten. Die Ausnehmungen 30a bis 30d können - müssen jedoch nicht - vormarkiert, vorgestanzt etc. sein.

Anstelle der Verwendung von aus Kunststoff gespritzten oder sonstwie gefertigten taschenartigen Aufnahmen, wie sie für z.B. Kunden- oder Scheckkarten bekannt sind, kann der Trägerkörper 4 auch aus einem zusammenhängenden langgestreckten Streifen aus z.B. steifem Karton oder auch aus Kunststoff gefertigt werden, wobei im letzteren Fall der Streifen in seiner Längserstreckungs-Mitte eine senkrecht zur Längserstreckung verlaufende Soll-Knickstelle aufweisen sollte. Der Streifen wird ausgeschnitten oder -gestanzt und mittig gefaltet. Es werden die laufende Nummer, eine Codenummer etc. überprüft und registriert und der Benutzername eingetragen und registriert. Sodann werden die benutzer- oder personenbezogenen Daten durch Entfernen oder dergl. der Aussparungen eincodiert, der nach der Faltung als zweischichtiger Körper aufgebaute Trägerkörper laminiert und danach die Laminierung gegenüber der Faltung aufgeschnitten, um den zwischen den beiden Hüllenteilen befindlichen durchgehenden Spalt oder Schlitz zu bilden, durch welchen in den Trägerkörper 4 eine Karte entsprechender Abmessung eingeschoben werden kann.

Anschließend wird noch geprüft, ob sich in den so gebildeten Trägerkörper eine Karte entsprechend der Karte 36 einführen läßt und die Codierung funktioniert, d.h. die Daten lesbar sind.

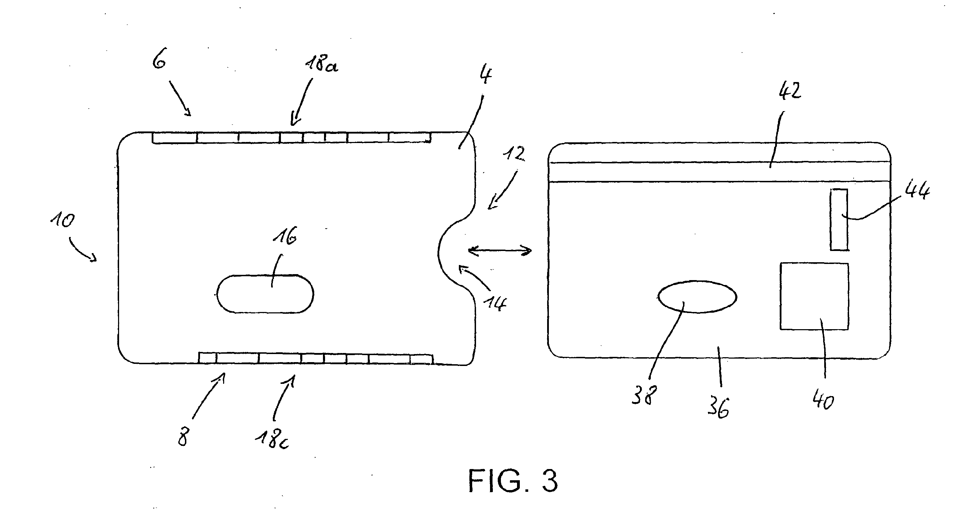

Fig. 3 zeigt eine weitere Ausgestaltungsform der vorliegenden Erfindung, bei der ein Trägerkörper 4 etwa gemäß Fig. 1 (ein Trägerkörper 4 der Ausführungsform von Fig. 2 ist natürlich gleichermaßen verwendbar) mit einer in diesen Trägerkörper 4 einschiebbaren Karte 36 (welche bevorzugt auch eine sogenannte - gegebenenfalls zu modifizierende - Krankenversichertenkarte ist) kombiniert wird. Der Trägerkörper 4 weist an seinen beiden Längsseiten 6 und 8 die Codierbereiche 18a und 18c auf (die Schmalseite 10 ist im Ausführungsbeispiel von Fig. 3 ohne Codierbereich; es versteht sich, daß ein derartiger Codierbereich entsprechend dem Codierbereich 18b von Fig. 1 vorgesehen werden kann).

An der Schmalseite 12 ist der Trägerkörper 4 schlitz- oder spaltförmig geöffnet, so daß die Karte 36 in den Trägerkörper 4 in bekannter Weise eingeschoben werden kann. Eine auf der Karte 36 aufgebrachte Identifikationsmarke 38 ist bei in den Trägerkörper 4 eingeschobener Karte 36 durch das Fenster 16 sichtbar und erlaubt eine Identifikation der Karte 36.

Die Karte 36 dient zunächst in bekannter Weise zur Erfüllung der bei einer Krankenversichertenkarte üblichen Funktionen und weiterhin zur Aufnahme weiterer Codiermöglichkeiten, das heißt zur Bereitstellung weiterer Möglichkeiten, fach- und/oder personenbezogene Daten abzuspeichern. Hierzu kann die Karte 36 in bekannter Weise einen auf oder im Trägerkörper integrierten Chip 40 und/oder Magnetstreifen 42 und/oder Transponder 44 aufweisen.

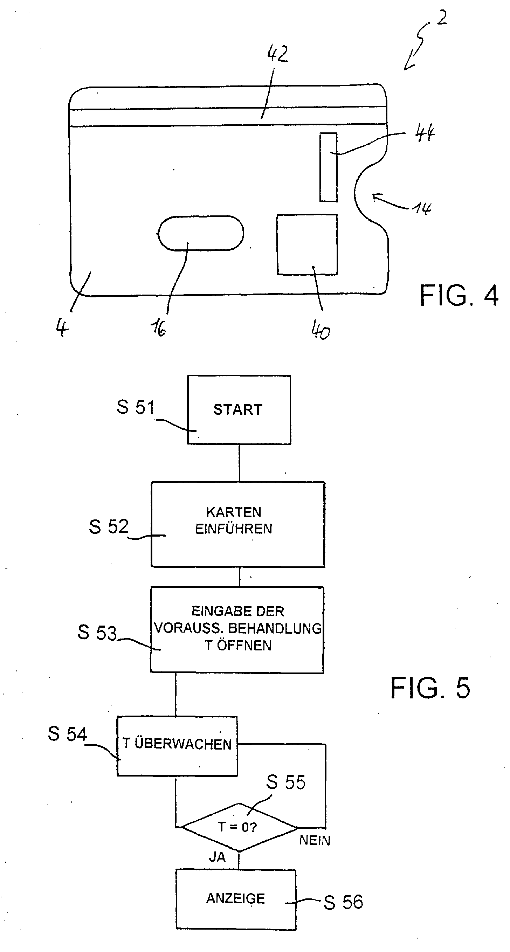

Fig. 4 zeigt eine weitere Ausführungsform der vorliegenden Erfindung, bei der die Ausführungsform von Fig. 3 mit derjenigen von Fig. 1 (oder Fig. 2) kombiniert wurde, nämlich derart, daß der Chip 40 und/oder der Magnetstreifen 42 und/oder der Transponder 44 auf bzw. in dem Trägerkörper 4 angeordnet sind. Der Trägerkörper 4 kann hierbei gemäß Fig. 4 ohne die Codierbereiche 18a bis 18c bzw. ohne die Ausnehmungen 30a bis 30d sein, oder aber er kann diese zusätzlichen Codiermöglichkeiten besitzen.

Es sei an dieser Stelle noch angemerkt, daß gruridsätzlich jede Art von Kombination der bisher beschriebenen und dargestellten Ausführungsformen möglich ist, also auch eine Kombination der Ausführungsformen gemäß den Figuren 1 und 2, das heißt sowohl mit umfangsseitig ausgebildeten Codierausnehmungen als auch sich direkt im Flächenmaterial des Trägerkörpers 4 befindlichen Codierausnehmungen.

Der als Schutzhülle für die (Krankenversicherten-)Karte 36 dienende Trägerkörper 4 erhält bevorzugt eine Knickschutzfunktion, mit der die Stabilität des Trägerkörpers - vor allem bei Schwächung des Trägerkörpers durch die Aussparungen und/oder Ausnehmungen - verbessert werden kann. Die Flexibilität soll dabei weitgehend erhalten bleiben, eine Abknickung hingegen, die zum Beispiel eintreten kann, wenn sich der Trägerkörper oder die Karte in Taschen von enganliegenden Hosen befindet und der Träger sich hinsetzt, soll verhindert werden.

Lösbar ist dies z.B. dadurch, daß an den (Längs-)Seiten 6 und 8 des Trägerkörpers 4 segmentierte Stränge mit einer unzerreißbaren Seele zum Beispiel aus Karbonfaser eingearbeitet werden, wobei mit dem Abstand der Segmente und der Stärke der Stränge die maximale Aus- oder Ablenkung, d.h. Durchbiegung eingestellt werden kann.

Hinsichtlich Einzelheiten eines derartigen segmentierten Stranges und seiner Funktions- und Wirkungsweise sei auf die PCT-Veröffentlichung PCT/EP00/01493 (WO00/50283) des gleichen Anmelders verwiesen; auf den dortigen Offenbarungsgehalt wird hier insofern vollinhaltlich Bezug genommen.

Die mit dem Gegenstand der vorliegenden Erfindung erzielbaren Vorteile, sowie weitere Einzelheiten und Aspekte des Erfindungsgegenstandes seien anhand der nachfolgenden, weiterhin als rein illustrativ zu verstehenden Beispiele näher erläutert, wobei als Einsatzbereich u.a., jedoch nicht ausschließlich das Gebiet der Dentalmedizin oder Dentaltechnik herangezogen sei.

### Beispiel 1:

Ein Zahnarzt stellt fest, daß neben seiner zahnärztlichen Tätigkeit der Patient den Therapiefortschritt durch Verwendung einer Zahncreme mit einer bestimmten Wirkstoffkombination unterstützen kann. Anstatt einer Empfehlung für eine bestimmte Zahncrememarke, woraufhin sich dann der Patient mit dieser Empfehlung in ein Geschäft, einen Drogeriemarkt oder dergleichen begibt, codiert der Zahnarzt oder eine mit dieser Aufgabe autorisierte Person aus seinem Praxispersonal die Information hinsichtlich der optimalen Wirkstoffkombination auf dem Datenträger des erfindungsgemäßen Informationsvermittlungssytems, wobei die weiter oben unter Bezugnahme auf die Figuren 1 bis 4 erläuterten Codierungsmöglichkeiten zur Anwendung gelangen können.

Mit dem Datenträger, auf welchem sich die notwendigen Informationen in unverlierbarer Form hinterlegt befinden, geht dann der Patient beispielsweise in einen Drogeriemarkt, wo der Datenträger, das heißt der Trägerkörper 4 und/oder die Karte 36 in ein entsprechendes Lesegerät eingeschoben werden. Das Lesegerät decodiert die enthaltenen Daten und zeigt auf einem Anzeigeschirm dasjenige Produkt bzw. diejenigen Produkte mit ihren dem Verbraucher geläufigen Produktnamen an, wobei dieses Produkt oder diese Produkte die vom Zahnarzt gewünschte Wirkstoffkombination enthalten. Der Anzeige-Bildschirm ist natürlich nur eine Möglichkeit der Produktempfehlung. Eine weitere Möglichkeit wäre z.B. die Hintergrundbeleuchtung der Aufhängevorrichtungen (Lochtafeln), Stapelvorrichtungen (Regale) für die Produkte, welche empfohlen werden.

Falls gewünscht, können auch noch Ausweichprodukte angezeigt werden, welche die vom Zahnarzt gewünschte Wirkstoffkombination beispielsweise nur in einem geringeren Prozentsatz enthalten, gleichwohl noch gegenüber anderen, willkürlich ausgewählten Produkten einen Therapievorteil bieten. Es bleibt nun dem Kunden überlassen, aus den ihm in Frage kommend dargestellten Produkten dasjenige auszuwählen, welches ihm hinsichtlich anderer Kriterien am meisten zusagt, also beispielsweise hinsichtlich Gebindegröße, Preis, Geschmack (falls es sich beispielsweise um eine Zahncreme, ein Mundwasser oder dergleichen handelt), Farbe (beispielsweise bei Zahnbürsten für Kinder) etc.

Wie bereits erwähnt, besteht eine andere Möglichkeit der Produktempfehlung darin, daß der Patient/Kunde im Drogeriemarkt die Karte 36 in ein Lesegerät steckt und dann, solange die Karte 36 steckt, auf der Verkaufswand das Feld oder die Felder (Regalfach oder -fächer) aufleuchten, die empfohlene Artikel enthalten. Dies setzt voraus, daß die Bestückung der Felder vordefiniert ist. Ein Feld enthält Artikel nur einer Produktgruppe aber von verschiedenen Herstellern und in verschiedenen Preisklassen.

In jedem Fall kann der Patient sicher sein, daß er - vorausgesetzt, er hat nicht eines der Ausweichprodukte gewählt - ein Produkt ersteht, welches neben den ihn persönlich befriedigenden Kriterien auch die von seinem Arzt geforderten Kriterien hundertprozentig erfüllt.

### Beispiel 2:

In der Ausführungsform von Fig. 3, das heißt bei der Kombination aus Trägerkörper 4 und Karte 36 werden vom Zahnarzt, vom Dentalhygieniker etc. die für die Kaufentscheidung von Mundhygiene-Artikeln wichtigen Parametern auf Seiten des Trägerkörpers 4 codiert, wobei entweder die Ausführungsform von Fig. 1 oder die Ausführungsform von Fig. 2 oder die Ausführungsform von Fig. 4 oder eine beliebige Kombination hieraus zur Anwendung gelangt. Der Chip 40, der Magnetstreifen 42 oder der Transponder 44 enthalten nach dem Vorbild von beispielsweise Telefonkarten einen bestimmten abrufbaren Geldbetrag, der gegen Erhalt der vollen Karte 36 beim Zahnarzt oder einer anderen Verkaufsperson zu entrichten ist.

Der Patient hat nunmehr in der Kombination aus Trägerkörper und Karte sowohl die notwendigen Informationen hinsichtlich der wichtigen Parameter der zu kaufenden Mundhygiene-Artikel, als auch die Möglichkeit, über die PrePaid-Karte 36 an der Ausgabestelle beim Erwerb der Mundhygiene-Artikel den fälligen Betrag einzulösen. Mit anderen Worten, der Betrag wird von der Karte 36 bzw. dem dortigen Chip 40, dem dortigen Streifen 42 oder dem dortigen Transponder 44 abgebucht. Sobald die Karte 36 "leer" ist, kann sie durch Entrichtung eines entsprechenden Betrages entweder beim Zahnarzt oder einer anderen autorisierten Person aufgeladen werden. Eine derartige Ausgabestelle für die Produkte muß nicht ausschließlich ein Drogeriemarkt etc. sein. Der Erfindungsgedanke bietet auch einen Weg zum unproblematischen Verkauf von (Hygiene)mitteln in der (Zahnarzt)praxis und erfüllt u.a. folgende, für den wirtschaftlichen Erfolg eines Praxisshops elementar wichtigen Kriterien:
geringstmögliche Belastung für das Praxispersonal;
automatische Warenabgabe;
Diebstahlsicherheit;
automatische Abrechnung;
einfache, systemintegrierte Lagerhaltung;
geringer Platzbedarf;
markenunabhängige Beratung und Verkauf;
therapiegebundener Verkauf;
therapieunterstützender Verkauf;
räumliche Trennung vom Praxisbetrieb; und
organisatorische Trennung vom Praxisbetrieb.

Zum System des Verkaufs in der Praxis gehört im Rahmen der Erfindung auch ein speziell entwickeltes Verkaufsmöbel, welches von einem entsprechenden Möbel z.B. in einem Drogeriemarkt abgeleitet sein kann oder identisch zu diesem sein kann.

### Beispiel 3:

Ist in der Ausführungsform gemäß Fig. 4 der Chip 40 oder der Magnetstreifen 42 oder der Transponder 44 nicht auf einer separaten Karte, sondern mit dem Trägerkörper 4 zu einer Einheit zusammengefaßt, ergeben sich im wesentlichen die gleichen Vorteile wie bei dem oben genannten Beispiel 2, wobei jedoch darüber hinaus keine separate Karte 36 erforderlich ist; der Trägerkörper 4 kann nach Art einer gewöhnlichen Chipkarten-Hülle beispielsweise zur Aufnahme einer Krankenkassenkarte verwendet werden.

### Beispiel 4:

Bei einer Kombination eines Trägerkörpers 4 in der Ausführungsform gemäß Fig. 4, das heißt mit dem Chip 40 und/oder dem Magnetstreifen 42 und/oder dem Transponder 44 mit einer Krankenversicherten- oder Krankenkassenkarte ergibt sich die folgende, als besonders vorteilhaft erachtete Einsatzmöglichkeit, welche unter Bezugnahme auf Fig. 5 näher erläutert werden soll: Zu Beginn der Behandlung beginnt automatisch ein Timer zu laufen. Beispielsweise schließt sich beim Setzen des Patienten in den Behandlungsstuhl ein Kontakt und dieser aktivierte Kontakt öffnet ein Zeitfenster. Dies ist in Fig. 5 mit dem Schritt S 51 veranschaulicht. Im darauffolgenden Schritt S 52 steckt der Zahnarzt in ein entsprechendes Lesegerät die Krankenversicherten-karte, welche zuvor aus dem Trägerkörper 4 gemäß Fig. 4 entnommen wurde, sowie den Trägerkörper 4 ein. In einem darauffolgenden Schritt S 53 gibt der Zahnarzt über eine Tastatur oder ein ähnliches Eingabegerät die voraussichtlichen Behandlungen ein, wodurch das Zeitfenster entsprechend erweitert wird. Die Länge dieses behandlungsrelevanten Zeitfensters ist unter anderem abhängig von dem aktuellen Punktwert der jeweiligen gesetzlichen Krankenkasse und der Größe des kontinuierlich abnehmenden Budgets (Gesamt- bzw. Sektoralbudget). Die dafür erforderlichen Daten werden On-Line ständig von der KZV aktualisiert. Der Ablauf des behandlungsrelevanten Zeitfensters mit dem Wert T wird in einem Schritt S 54 überwacht und in einem Schritt S 55 wird die Entscheidung getroffen, ob das Zeitfenster T abgelaufen ist oder nicht. Ist das Zeitfenster abgelaufen, wird im Schritt S 56 ein akustisches und/oder optisches Signal ausgegeben und der Zahnarzt und auch der Patient wissen jetzt, daß die Zeit der Privat-Liquidation beginnt und von der PrePaid-Karte, das heißt von dem Trägerkörper 4 abgebucht wird.

### Beispiel 5:

Die gleiche Funktion kann auch durch die Ausführungsform von Fig. 4 alleine durchgeführt werden, bei der die notwendigen Daten auf dem Chip 40 und/oder Magnetstreifen 42 und/oder Transponder 44 abgespeichert sind.

### Beispiel 6:

Eine weitere Möglichkeit, dem Benutzer die für die Kaufentscheidung von Mundhygieneartikeln wichtigen Parameter oder auch die erforderlichen Maßnahmen zur Durchführung einer adäquaten Mundpflege an die Hand geben zu können, ist, beispielsweise in der Kartencodierungsform von Fig. 1 eine Abbildung der Karte bzw. des Trägerkörpers 4 mit noch unversehrten Codierbereichen 18a, 18b und 18c bzw. in der Ausführungsform von Fig. 2 eine Abbildung des dortigen Trägerkörpers 4 mit noch nicht entfernten oder noch nicht geöffneten Ausnehmungen 30a bis 30d auf einer entsprechenden Seite des Internets darzustellen. Diese Seite kann entweder unter der Adresse des behandelnden Zahnarztes hinterlegt werden, oder aber sie ist - eine entsprechende Normung vorausgesetzt - für alle Zahnärzte verbindlich und steht in einer überregionalen Internetadresse zur Verfügung. Durch Vergleich und dann Anklicken der von seinem Zahnarzt entfernten Abschnitte 20a, 20b bzw. herausgestanzten oder herausgebrochenen Ausnehmungen 30a bis 30d (Übertragung der vom Arzt vorgenommen Codierung auf das Kartenmodell im Internet) erfolgt dann eine Verzweigung auf Unterseiten, wo die den entsprechenden Codierungen zugewiesenen Produktempfehlungen, Mundhygienemaßnahmen etc. im Volltext hinterlegt sind, so daß dem Patienten die entsprechenden Informationen übermittelt werden können. Diese Vorgehensweise hat den Vorteil, daß seitens des Handels keine entsprechenden Lesegeräte vorhanden sein müssen.

Eine andere Möglichkeit, das Internet im Rahmen der vorliegenden Erfindung zu nutzen, besteht darin, daß z.B. auf jeder der beiden Längsseiten der abgebildeten Karte 8 Felder (Buttons) zur Verfügung stehen. Wird auf jeder Längsseite nur ein Feld markiert, kann der Besucher/Kunde/Patient direkt auf die Ihn betreffende Internet-Seite mit den ihn betreffenden (Produkt-, Therapie-) Informationen gelotst werden. Zwar muß in diesem Fall eine digitale Rechenfunktion hinterlegt werden, aber diese Vorgehensweise erlaubt eine übersichtliche Darstellung auch wesentlich komplexerer Zusammenhänge.

Ein weiterer Vorteil ist in einer Steigerung der Übersichtlichkeit bei gleichzeitiger Platz- und Speicherplatz-Ersparnis zu sehen. Will man auf herkömmliche Weise den Besucher einer Homepage oder den Kunden eines Internet-Shops auf 64 Seiten mit je einer Produktgruppe lotsen, benötigt man 64 Buttons, durch die sich der Besuche/Kunde umständlich und zeitaufwendig hindurchsuchen muß.

Wie bereits weiter oben erläutert, ist der Gegenstand der vorliegenden Erfindung nicht nur im Verhältnis Zahnarzt/Patient, also das heißt auf dem Gebiet der Dentalmedizin oder Dentaltechnik anwendbar, sondern überall dort, wo auf dem medizinischen Sektor dem Patienten Informationen mitgegeben werden sollen, mit welchen er dann Produkte zu Therapieunterstützung bzw. entsprechende Verhaltensmaßnahmen mitgeteilt bekommt:

### Beispiel 7:

Eine weitere AnwendungsmMöglichkeit des Erfindungsgegenstandes ist im Bereich der Abrechnung mit gesetzlichen (und auch mit privaten) Krankenversicherern zu sehen. Dieses Thema hat durch die jüngsten Vorstöße in der Politik in letzter Zeit weiter an Aktualität gewonnen. So fordern Politiker aus dem Gesundheitswesen immer wieder, die Patienten sollten den Ärzten beim Verlassen der Praxis bestätigen, daß die abzurechnenden Leistungen auch wirklich erbracht worden sind. Um diese Forderung vor juristisch sauberem Hintergrund zu erfüllen, muß der Patient in die Lage versetzt werden, zunächst einmal unmißverständlich zu verstehen, was er dem Arzt bestätigen sollt. Denn der behandlende Arzt wird dem Patienten wohl kaum eine Aufstellung der gerade erbrachten Leistungen zur Bestätigung vorlegen dürfen, welche größtenteils aus für Laien unverständlichen Fachwörtern und Kürzeln besteht. Es wird somit nicht reichen, wenn der Arzt nach Beendigung der Behandlung erzwungenermaßen sofort in seine Abrechnungs-EDV die erbrachten Leistungen eingibt oder eingeben läßt und dem Patienten eine Kopie des dadurch erstellten Abrechnungsdatensatzes ausdruckt und zur Unterschrift übergibt. Abgesehen von dem gewaltigen Mehraufwand an Verwaltungsarbeit wird der Patient kaum verstehen, was er unterschreibt bzw. unterschreiben soll.

Im Sinne einer juristisch einwandfreien Vorgehensweise wird somit gefordert werden, daß der Patient eine allgemein verständliche Übersetzung der Fachausdrücke erhält; wenn der Arzt nicht sogar gezwungen wird, zusätzlich die letzten Unklarheiten noch mündlich aus dem Weg zu räumen. Die kassenärztlichen Bundes-Vereinigung hat daher bereits folgerichtig vorgeschlagen, dem Patienten gleich die "richtige" Rechnung auszuhändigen, die jener dann mit der Kasse direkt abrechnet. Dieses Vorgehen würde die Verwaltungsarbeit in den Praxen wesentlich erleichtern, allerdings mit dem Nachteil, daß dann die Kontroll-Funktion der Kassen(zahn)ärztlichen Vereinigungen entfällt und diese kostenintensive Aufgabe den Krankenkassen zufällt. Die meisten Patienten sind aber dennoch nicht in der Lage, Liquidationen innerhalb kurzer Zeit so gut zu verstehen, daß sie im Sinne der vom Bundes-Gesunheitsministerium geforderten Kontrolle quasi zwischen Tür und Angel handeln könnten.

Der Gegenstand der vorliegenden Erfindung bietet zu diesem Problem gemäß Beispiel 7 den Lösungsvorschlag, daß die Leistungen vom Arzt bzw. seiner Helferin in gewohnter Weise per EDV erfaßt werden. Bisher wurde am Ende des Quartals z.B. eine Diskette erstellt und an die Kassen(zahn)ärztliche Vereinigung geschickt, auf die nur Leistungspositionen gelangen konnten, welche auch das Prüfmodul passieren konnten.

Dieses Prüfmodul kann auch in Zukunft eingesetzt werden, und zwar so, daß die von der (gesetzlichen) Krankenkasse zu erstattenden Leistungen auf die Krankenversichertenkarte geschrieben werden und die darüber hinausgehenden Leistungen auf die Karte 36 und/oder den Trägerkörper 4 kopiert werden. Die Sprache dieser Daten kann durchaus weiterhin in Fachjargon (medizinische Fachausdrücke etc.) abgefaßt sein.

Zeitgleich kann eine automatisch per Software erstellte allgemeinverständliche Übersetzungs-Versionen der Kassen-Liquidation und der Privat-Liquidation ausgedruckt werden.

Zur Entlastung der Praxen kann auch ein Übersetzungs- und Druck-Paket (Lesegerät mit Software) allgemeinzugänglich auf dem Markt angeboten werden, so daß auf jedem Heim-PC der Inhalt von Krankenversichertenkarte und z.B. Trägerkörper 4 ausgelesen und ausgedruckt werden kann.

Zunächst ist der Patient Gesamtschuldner sowohl der Kassen- als auch der Privat-Liquidation. Der Patient kann den Schuldneranteil der Kassenliquidation dadurch loswerden, daß er sich
1. zur nächstgelegenen Niederlassung seiner Krankenkasse begibt, oder
2. der Krankenversichertenkarten-Abrechnungsdatensatz Online dorthin übermittelt wird, nämlich
   a) direkt von der Arztpraxis aus, oder
   b) vom Heim-PC aus.

Jede Krankenkassen-Zweigstelle hält zusätzlich zu einem Krankenversichertenkarten-Lesegerät ein Prüfmodul vorrätig, um ihrerseits die Abrechnungsdaten noch einmal auf Plausibilität und sachlich-rechnerische Richtigkeit prüfen zu können. Nach dieser Prüfung wird das Honorar direkt an den Arzt überwiesen.

In all diesen Fällen kann der Zweiteiligkeit der Anordnung von Krankenversichertenkarte (Karte 36) zum einen und der Abdeckung oder dem Trägerkörper (cover) 4 zum anderen eine besondere Kontroll-Funktion zukommen: Steckt der Patient nämlich Karte und Trägerkörper in bestimmter Weise (zum Beispiel einmal links herum und einmal rechts herum) zusammen, gilt dies als eine durch diesen Akt sowohl auf der Krankenversichertenkarte als auch auf dem Trägerkörper elektronisch gespeicherte Bestätigung der Gesamtabrechnung.

### Beispiel 8:

Bestimmte Behandlungsarten oder -formen, hierbei verwendete Techniken und Materialien etc. haben in den gültigen Gebührenordnungen, welche teilweise ein Jahrzehnt oder noch älter sind, keine oder nur unzureichende Abrechnungspositionen. Bedient sich ein Arzt einer neuzeitlichen, aktuellen Behandlung und/oder verwendet neuartige, weil bessere, Materialien, ist er bei der Abrechnung gezwungen, einschlägige Kommentare zu den einzelnen Gebührenordungen (GOZ, GOÄ etc.) zu Rate zu ziehen, in den empfehlungen angegeben sind welche Behandlung wie abgerechnet werden kann, auch wenn sie nicht in der zugehörigen Gebührenordnung steht.

Mit dem Gegenstand der vorliegenden Erfindung ist es (u.U. im Rahmen von oder in Kombination mit obigem Beispiel 7) möglich, neue Behandlungsmethoden und/oder -materialien seitens des Arztes, Technikers etc. auf dem Trägerkörper bzw. der Karte abzuspeichern und zentral, d.h. bei der zuständigen Kasse auf der Grundlage von bestehender Gebührenordnung und einschlägigen Kommentaren die tatsächliche Liquidation z.B. über eine entsprechende Software zu erstellen. Ein bestimmter Abrechnungsalgoritmus läßt sich ebenfalls zusätzlich abspeichern.

### Beispiel 9:

Z.B. im Bereich der Physiotherapie kann der Arzt bestimmte Behandlungsformen oder Übungen kodieren. Dies kann mit dem Gegenstand der vorliegenden Erfindung weitaus spezifizierter erfolgen als auf Rezepten, z.B. unter Angabe von Wiederholungszahl, Gewichtsbelastung etc.

Weiterhin ist der Gegenstand der vorliegenden Erfindung auch nicht auf medizinische/therapeutische Zwecke alleine beschränkt, sondern es ergeben sich noch weitere Anwendungsmöglichkeiten, z.B.:

### Beispiel 10:

Die spezifizierte Darstellung der physiotherapeutischen Behandlung läßt es sogar nahelegen, Patienten unter Kosteneinsparung in Fitnessstudios (statt beim Physiotherapeuten) kontrolliert zu behandeln. So ist folgendes Szenario vorstellbar: Der Patient steckt seine Karte in die Steuereinheit des Trainingsgerätes, wodurch die geforderte Übung mit den individuellen Parametern (z.B. Anstellwinkel, Gewichtsbelastung, Wiederholungszahl, Kraftverlauf, Dynamik etc.) eingestellt werden. Das Gerät wiederum kann den Übungsverlauf im Sinne eines Feedbacks und einer Verlaufskontrolle auf der Karte speichern. Dem Arzt und dem Kostenträger wird damit eine intensivere Kontrolle des Behandlungsverlaufs ermöglicht.

Genauso kann der Fitness-Trainer seinem Kunden einen Trainingsplan ausarbeiten und auf der Karte speichern. Die richtige Übungsausführung kann am jeweiligen Gerät dadurch dargestellt werden, daß die Karte automatisch einen Video-/CD-Clip auf einen Bildschirm ruft, der von der Realausführung überlagert wird und somit simultan Ausführungskontrolle ohne zwingende Anwesenheit des Trainers gewährleistet.

Darüberhinaus kann wiederum aus dem erzielten Kraftverlauf hochgerechnet und fundiert Einfluß auf die nächste Übungsreihe genommen werden, indem die zukünftigen Geräteinstellungen auf der Karte gespeichert werden. Der Kunde kann somit selbständig von Gerät zu Gerät gehen und hat seinen "Trainer" in Form der Karte immer dabei. Denkbar ist sogar, daß der Kunde zuhause an seinem PC seinen Trainingsverlauf verfolgen und evtl. ausdrucken kann.

### Beispiel 11:

Ein weiteres Anwendungsfeld ist auf dem Gebiet des Praxis-Managements zu sehen, beispielsweise zur Regelung der Benützung des praxiseigenen Parkplatzes: Der Absperrmechanismus, welcher unberechtigtes Parken verhindert, kann mit Hilfe der Karte/dem Trägerkörper, wie in einem Parkhaus, geöffnet werden. Um zu verhindern, daß der Parkplatz durch Patienten ständig besetzt ist oder von Patienten benützt wird, die sich nur sehr selten in Behandlung begeben bzw. die Prophylaxetermine nicht einhalten, wird jedem Quartal ein anderer Code zugeordnet. Eine Karte oder ein Trägerkörper bzw. die hierin enthaltenen Mittel (Transponder, Chip etc.) kann den Code von z.B. maximal drei Quartalen entziffern, so daß der Patient, der halbjährlich zur Kontroll-Untersuchung kommt, auch eine Parkberechtigung behält.

### Beispiel 12:

Weiterhin ist eine Speicherung des nächsten (Zahn-) Arzttermines auf der Karte oder dem Trägerkörper möglich; dies erspart dem Patienten die Aufbewahrung und vor allem die Suche nach Terminzetteln etc. Die KVK und Termininformationen sind immer zusammen. An die Möglichkeit eines zusätzlichen Aufmerksam-Machens auf den bevorstehenden Termin mit Hilfe von Blink- oder akustischen Signalen sei hiermit ebenfalls hingewiesen. Ihrer Realisierung steht aufgrund der fortschreitenden Miniaturisierung auf dem Bereich der Elektronik nichts im Wege.

Als Beispiel sei nur eine pop-up-Nachricht (oder mehrere, zeitlich gestaffelte Nachrichten mit zunehmender Dringlichkeit) auf dem privaten PC oder LapTop genannt, der mit einem Lesemodul die Termindaten eingespeichert erhalten hat.

### Beispiel 13:

Die Kombination Krankenversicherten-Karte mit Trägerkörper (cover) erlaubt auch eine Kontrolle über Missbrauch der Krankenversicherten-Karte. Wie schon erwähnt, kann der Patient entscheiden, wie er behandelt werden will: Gibt er nur die Krankenversicherten-Karte ab, bringt er zum Ausdruck, daß er nicht zuzahlen will und nur Kassenleistungen erbracht haben will. Gibt er die Krankenversicherten-Karte mit/in dem Trägerkörper ab, dokumentiert er, daß er zwar gesetzlich krankenversichert ist, aber weitergehende Leistungen über den Trägerkörper privat abgerechnet haben will. Gibt er den Trägerkörper alleine ab, bedeutet dies, daß er keine gesetzliche Krankenversicherung besitzt und die Abrechnung rein privat erfolgen soll.

Jedoch gibt mancher Patient seine Krankenversicherten-Karte unabsichtlich oder wissentlich nicht mit Beendigung des Versicherungsverhältnisses an die Krankenkasse zurück, sondern sucht weiterhin Ärzte auf. Diese behandeln im Vertrauen auf bestehenden Versicherungschutz und merken erst Monate später aufgrund von Regressverfahren via Kassen(zahn)ärztliche Vereinigung, daß sie unentgeltlich behandelt haben. Der bereits beschriebene Weg der Abrechnung, bei dem der Patient zunächst das Gesamthonorar schuldet und den Kassenanteil entrichtet, indem online mit der Kasse vor Ort oder direkt in der Zweigstelle die Forderung abgetreten wird, schließt diesen Missbrauch aus.

Soweit zu einzelnen, als nicht einschränkend zu verstehenden Beispielen.

Die Hardware-Komponente der vorliegenden Erfindung (Trägerkörper bzw. Kartenaufnahme 4) verdeutlicht symbolhaft das ganze erfindungsgemäße Konzept: Die Krankenversicherungskarte (KVK) wird von dem Trägerkörper 4 umhüllt und geschützt. Die KVK bildet das "Herz" des ganzen Systems, sie gibt alle versicherungsbezogenen Personendaten an das System ab. Der Trägerkörper erweitert den Schutz, auf ihm werden alle gesundheitsbezogenen Personendaten gespeichert und bei Bedarf an das System weitergegeben. Beide zusammen ermöglichen die erweiterte Honorargestaltung.

Durch den Gegenstand der vorliegenden Erfindung lassen sich somit u.a. die folgenden Aspekte realisieren bzw. Vorteile erzielen, wobei die nachfolgende Aufstellung ebenso wie die voranstehenden Beispiele als nicht ausschließlich oder einschränkend zu verstehen sind:
- Die von den Versicherungsträgern bezahlte Behandlungszeit läßt sich erfassen und dem Patienten die benötigte Restzeit in Rechnung stellen.
- Es handelt sich beim Erfindungsgegenstand um ein System, auf das der Patient schon im Vorhinein eingeht oder auch nicht.
- Ohne lange Diskussion herrscht Klarheit darüber, ob der Patient zuzahlen will. Der Arzt kann sich darauf einstellen. Der Patient, durch das akustische/optische Signal, welches das Ende der kassenbezahlten Behandlungszeit ankündigt, sensibilisiert, kann den Sinn des Systems und den Unsinn des budgetierten Kassen-Systems leichter erfassen. Er wird in seiner Entscheidung bestärkt oder kann sich im Nachhinein immer noch dazu entschließen, sich auf eine weiterführende Behandlung einzulassen.
- Durch den Kauf der prePaid-Karte hat der Patient schon von vorneherein seine Bereitschaft zur Zuzahlung bekundet, weshalb auch in diesem Punkt Rechtsklarheit herrscht.
- Durch die Höhe des prePaid-Betrages kann er dem Arzt auch Grenzen in der Behandlung vorgeben.
- Gibt der Patient nur die KVK ab, weiß der (Zahn-)Arzt, daß nur die Basisversorgung gewünscht wird. Gibt der Patient beide zusammen ab, kann die Behandlung unter den Bedingungen der erweiterten Honorargestaltung erfolgen. Aus dem KVK-Chip wird somit die Bezahlung der Basisversorgung gezogen.
- Gleichzeitig - oder auch nicht - wird aus dem Trägerkörper die Zuzahlung, abgerechnet nach dem Maß der Überschreitung der budgetierten Kasssenzeit, gezogen.
- Die Speicherung von gesundheitspezifischen Daten auf dem Trägerkörper ermöglicht auch den schon lange geforderten Transfer von einem Praxis-Computer zum anderen, wobei der Patient - und das ist von besonderer Wichtigkeit - die Hoheit über seine Daten behält und nicht wie in verschiedenen, von Krankenkassen und Gesundheitspolitikern bereits vorgestellten Zukunfts-Systemen, die Entscheidung, wem er seine Gesundheitsdaten gibt, an ein von Krankenkassen beherrschtes Datenzentrum abtritt. Schließlich muß nicht jeder Angestellte irgendeiner Krankenkasse und nicht irgendeine Arzthelferin schon beim ersten Kontakt mit dem Patienten in dessen intimsten Angelegenheiten Bescheid wissen.
- Die auf der Karte und/oder dem Trägerkörper gespeicherten bzw. speicherbaren Daten müssen sich nicht auf die Themenbereiche Mundhygiene und/oder Abrechnung beschränken. Ebenso können auch Daten beispielsweise über Materialien gespeichert werden, die bei der Behandlung verwendet wurden (in Erfüllung der Auflagen des Medizinproduktgesetzes, MPG), sowie Daten über für die zahnärztliche Behandlung relevante Allgemeinerkrankungen (Allergien, Medikamenteinnahmen etc.).
- Das Prüfmodul aus obigem Beispiel 7 kann weiterhin unter der Kontrolle der KZV stehen und dessen Inhalt Gegenstand von Verhandlungen zwischen KZV und Kassen bleiben. Verhandlungsergebnisse mit geringerem Zahlungsvolumen der Kassen gehen dann nicht mehr zu Lasten der Leistungserbringer, sondern aufgrund des Trägerkörper-KVK-Systems zu Lasten der Patienten, die zugleich Kassen-Mitglieder und zahlenmäßig von größerer Bedeutung als die Leistungserbringer sind und somit größeres politisches Gewicht ins Verhandlungsgefüge einbringen.

Beschrieben wurde ein Informationsvermittlungssystem, mit welchem wenigstens eine erste, fachlich autorisierte Person fach- und/oder personenbezogene Daten, welche wenigstens einer anderen Person zugeordnet sind, in codierter und von dieser Person mitführbarer Form aufzeichnen und festhalten kann, um es dieser wenigstens einen anderen Person und/oder wenigstens einer weiteren, ebenfalls fachlich autorisierten Person zu ermöglichen, ohne persönliche Anwesenheit der ersten autorisierten Person anhand der fach- und/oder personenbezogenen Daten Handlungsentscheidungen zu treffen. Die Daten sind auf einem von der Person mitführbaren Datenträger in unverlierbarer Form hinterlegt, wobei die Daten auf dem Datenträger durch wenigstens eine, bevorzugt mehrere das Material des Datenträger durchlässig machende Ausnehmungen in Form von umfangsseitig ausgebildeten, zahnförmigen Aussparungen codiert sind. Gegenstand der Erfindung ist weiterhin die Verwendung eines derartigen Informationsvermittlungssystems durch Ärzte, Therapeuten, Medizintechniker, Dentalhygieniker etc. und zur Mitgabe der so abgespeicherten Daten an eine diese Daten betreffende Person, welche diese Daten dann für sich selbst oder durch eine andere fachlich autorisierte Person auswerten kann bzw. ein Verfahren mit einem derartigen Informationsvermittlungssystem.

## Patentansprüche

1. Informationsvermittlungssystem, mit welchem wenigstens eine erste, fachlich autorisierte Person fach- und/oder personenbezogene Daten, welche wenigstens einer anderen Person zugeordnet sind, in codierter und von dieser Person mitführbarer Form aufzeichnen und festhalten kann, um es dieser wenigstens einen anderen Person und/oder wenigstens einer weiteren, ebenfalls fachlich autorisierten Person zu ermöglichen, ohne persönliche Anwesenheit der ersten autorisierten Person anhand der fach- und/oder personenbezogenen Daten Handlungsentscheidungen zu treffen.

2. System nach Anspruch 1, **dadurch gekennzeichnet, daß** die Daten auf einem von der Person mitführbaren Datenträger (2) in unverlierbarer Form hinterlegt sind.

3. System nach Anspruch 2, **dadurch gekennzeichnet, daß** der Datenträger (2) in Form eines flächigen Trägerkörpers (4) ausgebildet ist.

4. System nach Anspruch 3, **dadurch gekennzeichnet, daß** der Trägerkörper (4) festgelegte Abmessungen aufweist, insbesondere die Abmessungen einer üblichen Schutzhülle für Scheckkarten hat.

5. System nach Anspruch 3 oder 4, **dadurch gekennzeichnet, daß** die Daten auf dem Trägerkörper (4) mittels wenigstens einen im Trägerkörper integrierten Chip (40) und/oder Magnetstreifen (42) und/oder Transponder (44) abspeicherbar sind.

6. System nach Anspruch 3 oder 4, **dadurch gekennzeichnet, daß** die Daten auf dem Trägerkörper (4) durch wenigstens eine, bevorzugt mehrere das Material des Trägerkörpers durchtretende Ausnehmungen codiert sind.

7. System nach Anspruch 6, **dadurch gekennzeichnet, daß** die Ausnehmungen in Form von umfangsseitig am Trägerkörper (4) ausgebildeten, zahnförmigen Aussparungen (20a, 20b, 22, 24) gebildet sind.

8. System nach Anspruch 6, **dadurch gekennzeichnet, daß** die Ausnehmungen in Form von in der Fläche des Trägerkörpers (4) ausgebildeten Durchbrechungen (30a - 30d) gebildet sind.

9. System nach einem der Ansprüche 6 bis 8, **dadurch gekennzeichnet, daß** die Ausnehmungen in zweckorientierten Gruppen und/oder Reihen und/oder Spalten angeordnet sind.

10. System nach einem der Ansprüche 6 bis 9, **dadurch gekennzeichnet, daß** die Ausnehmungen durch Herausbrechen und/oder Herausstanzen und/oder Herausschneiden aus dem Material des Trägerkörpers (4) bildbar sind.

11. System nach Anspruch 10, **dadurch gekennzeichnet, daß** die Stellen, an welchen die Ausnehmungen ausbildbar sind, durch Markierungen in Form von Vorstanzungen und/oder Linien und/oder Farbflächen vorgegeben sind.

12. System nach einem der Ansprüche 4 bis 11, **dadurch gekennzeichnet, daß** in den als Schutzhülle ausgebildeten Trägerkörper (4) eine zum System gehörende Karte (36) einführbar und hierin aufbewahrbar ist.

13. System nach Anspruch 12, **dadurch gekennzeichnet, daß** auf der Karte (36) zusätzliche fachbezogene Daten, welche der anderen Person zugeordnet sind, abspeicherbar sind.

14. System nach Anspruch 13, **dadurch gekennzeichnet, daß** die Daten auf einem in der Karte (36) integrierten Chip (40) und/oder Magnetstreifen (42) und/oder Transponder (44) abspeicherbar sind.

15. Verwendung eines Informationsvermittlungssystems nach wenigstens einem der Ansprüche 1 bis 14 zur Abspeicherung fach- und/oder personenorientierter Daten in codierter Form und zur Mitgabe der so abgespeicherten Daten an eine diese Daten betreffende Person, welche diese Daten dann für sich selbst oder durch eine andere fachlich autorisierte Person auswerten kann.

16. Verwendung eines Informationsvermittlungssystems nach wenigstens einem der Ansprüche 1 bis 14 zur Abspeicherung fach- und/oder personenorientierter Daten in codierter Form durch einen Arzt, Therapeuten, Medizintechniker, Dentalhygieniker oder eine sonstige, auf dem medizinischen Sektor arbeitende und/oder geschulte, fachlich autorisierte Person und zur Mitgabe der so abgespeicherten Daten an eine diese Daten betreffende Person, welche diese Daten dann für sich selbst oder durch eine andere fachlich autorisierte Person auswerten kann.

17. Verfahren zur Informationsübermittlung und -auswertung, bei welchem:
fach- und/oder personenorientierte Daten durch eine fachlich autorisierte Person in codierter Form abgespeichert werden; und
die so abgespeicherten Daten einer diese Daten betreffenden Person mitgegeben werden, welche dann
diese Daten dann für sich selbst oder durch eine andere fachlich autorisierte Person auswertet.

18. Verfahren zur Informationsübermittlung und -auswertung, bei welchem:
fach- und/oder personenorientierte Daten durch einen Arzt, Therapeuten, Medizintechniker, Dentalhygieniker oder eine sonstige, auf dem medizinischen Sektor arbeitende und/oder geschulte, fachlich autorisierte Person in codierter Form abgespeichert werden; und
die so abgespeicherten Daten einer diese Daten betreffenden Person mitgegeben werden, welche dann
diese Daten dann für sich selbst oder durch eine andere fachlich autorisierte Person auswertet.
